(19) Europäisches Patentamt
European Patent Office
Office européen des brevets

(11) Publication number: **0 351 597**
**A2**

(12) **EUROPEAN PATENT APPLICATION**

(21) Application number: **89111730.1**

(22) Date of filing: **28.06.89**

(51) Int. Cl.⁴: **C07K 9/00 , C07K 7/06 , C07K 1/00 , A61K 37/02**

(30) Priority: **21.07.88 GB 8817397**

(43) Date of publication of application: **24.01.90 Bulletin 90/04**

(84) Designated Contracting States: **AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Malabarba, Adriano**
**5/A, Via Roma**
**I-20082 Binasco (MI)(IT)**
Inventor: **Trani, Aldo**
**11, Via Longhi**
**I-20137 Milano(IT)**

(54) **"N15-alkyl and N15,N15-di-alkyl derivatives of teicoplanin antibiotics carrying functional groups on the alkyl side chain.**

(57) The present invention relates to new N-monoalkyl and N-dialkyl derivatives of teicoplanin carrying functional groups on the alkyl chain.

The compounds of the invention can be prepared according to a variety of alkylation of a teicoplanin substrate such as an alkylation with an alkyl halide in the presence of a base or a reductive alkylation with a carbonyl compound in the presence of an alkali metal borohydride.

The compounds show good antibacterial activity mainly against gram-positive bacteria.

EP 0 351 597 A2

## $N^{15}$-ALKYL AND $N^{15},N^{15}$-DI-ALKYL DERIVATIVES OF TEICOPLANIN ANTIBIOTICS CARRYING FUNCTIONAL GROUPS ON THE ALKYL SIDE CHAIN

$N^{15}$-alkyl and $N^{15},N^{15}$-di-alkyl derivatives of teicoplanin compounds having the following formula:

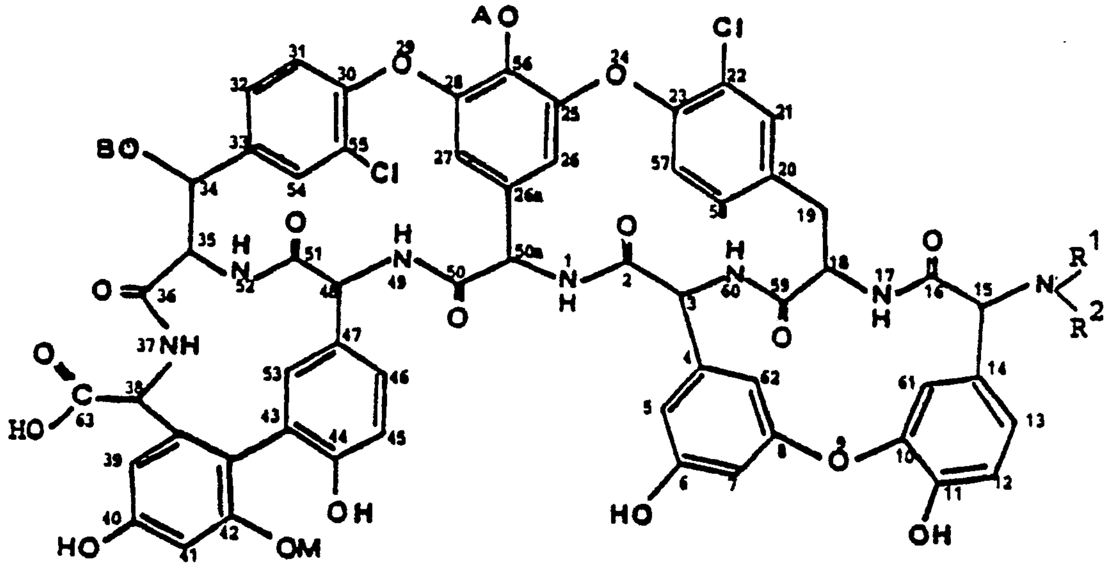

I

wherein:
$R^1$ represents a group
$[CHR^3(CR^4R^5)_mX]_p-(CH_2)_n-R^6$ II
wherein $R^3$ and $R^4$ independently represent H or a $(C_1-C_6)$ alkyl; $R^5$ represents H, a $(C_1-C_6)$alkyl, OH; $R^6$ represents H, $COOR^7$, $SR^7$; $NR^7R^8$, $OR^7$, halogen or a $(C_1-C_3)$alkyl; $R^7$ and $R^8$ independently represent H, a $(C_1-C_3)$alkyl; m, n and p are integers wherein m is zero or 1, $0 \leq n \leq 6$, $1 \leq P \leq 6$; X is O, NH or a bond with the proviso that when X is O or NH, $n \neq 0$, $1 \leq p \leq 3$, and $R^5$ is different from OH; $R^2$ represents hydrogen or a $(C_1-C_3)$alkyl group; with the proviso that $R^1$ must not represent a $(C_1-C_{12})$alkyl group
A represents hydrogen or -N[$(C_9-C_{12})$aliphatic acyl]-beta-D-2-deoxy-2-amino-glucopyranosyl;
B represents hydrogen or N-acetyl-beta-D-2-deoxy-2-amino-glucopyranosyl;
M represents hydrogen or alpha-D-mannopyranosyl; with the proviso that B represents hydrogen only when A and M are simultaneously hydrogen; and the addition salts thereof,

Teicoplanin is the international non-proprietary name (INN) of the antibiotic substance formerly named teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751).

According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures.

Teichomycin $A_2$, which is the major factor of the isolated antibiotic complex, is then separated from the other factors by means of column chromatography on Sephadex$^R$.

British Patent No. 2121401 discloses that antibiotic Teichomycin $A_2$ actually is a mixture of five closely related co-produced main components.

It is possible to represent teicoplanin $A_2$ (formerly Teichomycin $A_2$) main components 1, 2, 3, 4 and 5 by the above formula I wherein $R^1$ and $R^2$ are replaced by hydrogen atoms, A represents -N[$(C_{10}-C_{11})$-aliphatic acyl]-beta-D-2-deoxy-2-amino-glucopyranosyl, B represents N-acetyl-beta-D-2-deoxy-2-amino-glucopyranosyl, M represents alpha-D-manno-pyranosyl.

More particularly, in teicoplanin $A_2$ component 1, the [$(C_{10}-C_{11})$-aliphatic acyl] substituent represents Z-4-decenoyl, in teicoplanin $A_2$ component 2 represents 8-methyl-nonanoyl, in teicoplanin $A_2$ component 3

represents decanoyl, in teicoplanin $A_2$ component 4 represents 8-methyldecanoyl, in teicoplanin $A_2$ component 5 represents 9-methyldecanoyl.

European Patent Application Ser. No. 88110194 describes production of teicoplanin compounds where the aliphatic acyl group of the beta-D-2-deoxy-2-amino-glucopyranosyl moiety is a N-nonanoyl (compund B or RS3) or a 6-methyl octanoyl group (compound A or RS4).

In the paper entitled: "Isolation by HPLC and structural determination of minor components of teicoplanin" given by Zanol et al., at the 17th International Symposium on chromatography, Wien, September, 25-30, 1988, other two teicoplanin compounds (RS1 and RS2) are described.

Said compounds are characterized in that the aliphatic acyl moieties of the beta-D-2-deoxy-2-amino-glucopyranosyl moiety are respectively methyl-undecanoyl and dodecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties.

They are named antibiotic L 17054 and antibiotic L 17046 and are described in European Patent Application Publication No. 119575 and European Patent Application Publication No. 119574, respectively.

Preferred hydrolysis conditions for the production of antibiotic L 17054 are: 0.5 N hydrochloric acid at a temperature between 70°C and 90°C and for a time which is generally between 15 and 90 min.

Antibiotic L 17054 is represented by the above formula I wherein $R^1$, $R^2$ and A represent hydrogen, B represents N-acetyl-beta-D-2-deoxy-2-amino-glucopyranosyl, M represents alpha-D-mannopyranosyl wherein the sugar moieties are linked to the peptidic nucleus through an O-glycosidic bond.

Preferred hydrolysis conditions for the preparation of antibiotic L 17046 are: 1-3 N hydrochloric acid, at a temperature between 50° and 90°C and for a time which is generally between 30 and 60 min.

Antibiotic L 17046 is represented by the above formula I wherein $R^1$, $R^2$, A and M represent hydrogen atoms, and B is N-acetyl-beta-D-2-deoxy-2-aminoglucopyranosyl wherein the sugar moiety is linked to the peptidic nucleus through an O-glycosidic bond.

European Patent Application Publication No. 301247 describes de-mannosyl teicoplanin derivatives i.e. compounds of the formula I above wherein A and B are different from hydrogen and M is hydrogen.

The complete selective cleavage of all the sugar moieties of the teicoplanin compounds gives an aglycone molecule which is called antibiotic L 17392, or deglucoteicoplanin, and is represented by the above formula I wherein $R^1$, $R^2$, A, B, and M each individually represents a hydrogen atom. This selective hydrolysis process is described in European Patent Application Publication No. 146053.

A substance having the same structural formula is disclosed in European Patent Application Publication No. 0090578 and is named antibiotic A 41030 factor B.

This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392 (compound A or RS3 compound B or RS4, RS1, RS2, the de-mannosyl derivatives of European Patent Application Publication No. 301247 and any mixture thereof in any proportion, are suitable starting materials for the preparation of the alkyl derivatives of the invention.

In the present specification "teicoplanin compound" or "teicoplanin starting material" is used to indicate any one of the above starting materials, i.e. teicoplanin as obtained according to U.S. patent 4,239,751, any further purification thereof, teicoplanin $A_2$ complex, a compound of the above formula I wherein $R^1$ and $R^2$ are hydrogen, A represents hydrogen or -N[($C_9$-$C_{12}$)aliphatic acyl]-beta-D-2-deoxy-2-amino-glucopyranosyl, B represent hydrogen or N-acetyl-beta-D-2-deoxy-2-amino-glucopyranosyl, M represents hydrogen or alpha-D-mannopyranosyl, with the proviso that B may represent hydrogen only when A and M are simultaneously hydrogen, a salt thereof, or a mixture thereof in any proportion.

As used herein the term "alkyl", either alone or in combination with other substituents, includes both straight and branched hydrocarbon groups; more particularly, "($C_1$-$C_6$)alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexanyl, 2-hexanyl, 3-hexanyl, 3,3-dimethyl-1-butanyl, 4-methyl-1-pentanyl and 3-methyl-1-pentanyl; likewise, "($C_1$-$C_4$)alkyl" represents a straight or branched hydrocarbon chain of 1 to 4 carbon atoms like those exemplified above.

The term "halogen" represents a halogen atom selected from fluorine, chlorine, bromine and iodine.

The compounds of the invention contain acidic and basic functions and can form, in addition to "internal salts", acid and base addition salts according to conventional procedures.

For the scope of the present description and claims the "internal salts" are encompassed by the definition of "non-salt" or "non-addition salt" form.

Preferred addition salts are the pharmaceutically acceptable acid and/or base addition salts.

With the term "pharmaceutically acceptable acid and/or base addition salts" are intended those salts with acids and/or bases which from biological, manufacturing and formulation standpoint are compatible with the pharmaceutical practice as well as with the use in the animal growth promotion.

Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, aspartic, phthalic, tartaric, lauric, stearic, salicylic, methanesulfonic, benzenesulfonic, sorbic, picric, benzoic, cinnamic and the like acids and salts with aminoacids like lysine, arginine, asparagine, glutamine, hystidine, serine, methionine, leucine and the like.

Representative examples of these bases are: alkali metal or alkaline-earth metal hydroxide such sodium, potassium, and calcium hydroxide; and organic aliphatic, alicyclic or aromatic amines such as methylamine, dimethylamine, trimethylamine, and picoline.

The N-alkyl and N,N-di-alkyl derivatives of teicoplanin compounds, according to the present invention are characterized by carrying functional groups on the alkyl side chain. It was found that the teicoplanin antibiotic substances having said $N$-$^{15}$ (and $N^{15}$,$N^{15}$-di-alkyl derivatives) show good antibacterial properties and permit an improved solubility of the antibiotic in an aqueous medium so obtaining more practical pharmaceutical compositions.

A preferred group of compounds of the invention is represented by those compounds of formula I wherein $R^1$ represents a group

$[CHR^3 (CR^4R^5)_mX]_p$- $CH_2)_n$-$R^6$

wherein $R^3$ and $R^4$ independently represents H or a $(C_1$-$C_4)$alkyl; $R^5$ represents H, a $(C_1$-$C_4)$alkyl or OH; X is NH, O or a bond, with the proviso that when X is O or NH, $n \neq 0$ and $R^5$ is different from OH, $R^6$ represents H, $COOR^7$, $S(R^7)$, $N(R^7R^8)$, $OR^7$, halogen or a $(C_1$-$C_3)$alkyl; $R^7$ and $R^8$ independently represents H or a $(C_1$-$C_3)$alkyl, m, n and p are integers wherein m is zero or 1, $0 \leq n \leq 3$, $1 \leq p \leq 3$ and $R^2$ represents hydrogen or a $(C_1$-$C_3)$alkyl; with the proviso that $R^1$ must not represent a $(C_1$-$C_{12})$alkyl.'

Another preferred group of compounds of the invention is represented by those compounds which belong to formula I wherein $R^1$ represents a group in which $R^3$ and $R^4$ independently represents H or a $(C_1$-$C_4)$alkyl; $R^5$ represents H, a $(C_1$-$C_4)$alkyl or OH; X is O or a bond; $R^6$ represents H, $(C_1$-$C_3)$alkyl, $S(R^7)$, $N$-$(R^7R^8)$, $OR^7$; $R^7$ and $R^8$ independently represent H or a $(C_1$-$C_3)$alkyl; m, n and p are integers wherein m is zero or 1, $1 \leq n \leq 3$, $1 \leq p \leq 3$ and $R^2$ is H or a $(C_1$-$C_3)$alkyl.

A futher preferred group of compounds of the present invention comprises the compounds of formula I wherein $R^6$ represents $N(R^7R^8)$ or $OR^7$ and A, B and M represent the sugar moieties defined above or A, B and M represent hydrogen atoms.

Preferred compounds of the invention are those compounds of formula I wherein A, B and M represents the sugar moieties defined above or A, B and M represent hydrogen atoms. Further preferred compounds are those compounds of formula I wherein A, B and M represents the sugar moieties defined above and the $N[(C_9$-$C_{12})$aliphatic acyl substituent represents 8-methylnonanoyl.

Another preferred group of compounds of the invention is represented by those compounds of formula I wherein $R^1$ represents a group in which n ranges from 0 to 3, p ranges from 1 to 3, $R^6$ is $OR^7$, m is zero or 1, X is O, or a bond, $R^3$ and $R^4$ represent a H or $(C_1$-$C_6)$alkyl group, $R^5$ is H, a $(C_1$-$C_6)$alkyl, or OH, $R^2$ is H or a $(C_1$-$C_3)$alkyl, and $R^7$ is as defined above.

Another preferred group of compound of the invention is represented by those compounds of formula I wherein $R^1$ represents a group in which n ranges from 0 to 3, p ranges from 1 to 3, m is zero or 1, $R^3$ and $R^4$ represent a $(C_1$-$C_6)$alkyl group or H; X is NH or a bond, $R^5$ is H, a $(C_1$-$C_6)$alkyl or OH, $R^6$ is $N(R^7R^8)$, $R^2$ is H or a $(C_1$-$C_3)$alkyl, and $R^7$ and $R^8$ are as defined above.

Other preferred compounds of the invention are:

a compound of formula I wherein $R^1$ represents a group in which $R^3$ and $R^4$ independently represent H or $CH_3$; $R^5$ represents H or OH; $R^6$ represents H, $OR^7$ or $NR^7R^8$; $R^7$ and $R^8$ independently represent H or $CH_3$; m is zero or 1, n is zero or 1, p is 1, X is O or a bond with the proviso that when X is O, n is 1 and $R^5$ is H or $CH_3$

- a compound of formula I wherein $R^1$ represents:

$-(CH_2)_p\ SCH_3$;

$-(CH_2)_p$ SH;
$-(CH_2)_p$ $COOCH_3$;
$-(CH_2)_p$ COOH;
$-(CH_2)_p$ $NH_2$;
$-(CH_2)_p$ $N(CH_3)_2$;
wherein $1<p<6$, and $R^2$ represents H or a $(C_1-C_3)$alkyl.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.

For instance, a compound of formula I can be transformed into the corresponding acid or base addition-salt by dissolving the non-salt form in an aqueous solvent and adding a slight molar excess of the selected acid or base.

The resulting solution or suspension is then lyophilized to recover the desired salt. Instead of lyophilizing, in some instances, it is possible to recover the final salt by extraction with an organic solvent, concentration to a small volume of the separated organic phase and precipitation by adding a non-solvent.

In case the final salt is insoluble in an organic solvent where the non-salt form is soluble it is recovered by filtration from the organic solution of the non-salt form after addition of the stoichiometric amount or a slight molar excess of the selected acid or base.

The non-salt form can be prepared from a corresponding acid or base salt dissolved in an aqueous solvent which is then neutralized to free the non-salt form.

This is then recovered for instance by extraction with an organic solvent or is transformed into another base or acid addition salt by adding the selected acid or base and working up as above.

When following the neutralization desalting is necessary, a common desalting procedure may be employed.

For example, column chromatography on controlled pore polydextrane resins (such as Sephadex L H 20) or silanized silica gel may be conveniently used.

After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique.

After formation and isolation, the salt form of a compound of formula I can be transformed into the corresponding non-salt or into a pharmaceutically acceptable salt.

In some instances the acid addition salt of a compound of formula I is more soluble in water and hydrophilic solvents and has an increased chemical stability.

However, in view of the similarity of the properties of the compounds of formula I and their salts, what is said in the present application when dealing with the biological activities of the compounds of formula I applies also to their pharmaceutically acceptable salts, and viceversa.

The compounds of the invention are useful as semi-synthetic antibacterial agents mainly active against gram positive bacteria. They also fall to be considered as growth promoters and their formulation and use for this purpose are within the scope of this invention.

The mono- and di-alkyl derivatives of the invention can be prepared according to a variety of alkylation procedures depending on the desired alkyl subtituents such as an alkylation with an alkyl halide in the presence of a base or a reductive alkylation with a carbonyl compound in the presence of an alkali metal borohydride.

A preferred procedure for preparing a mono-alkyl derivative of the invention is represented by an alkylation of a teicoplanin compound as defined above or a compound of formula III.

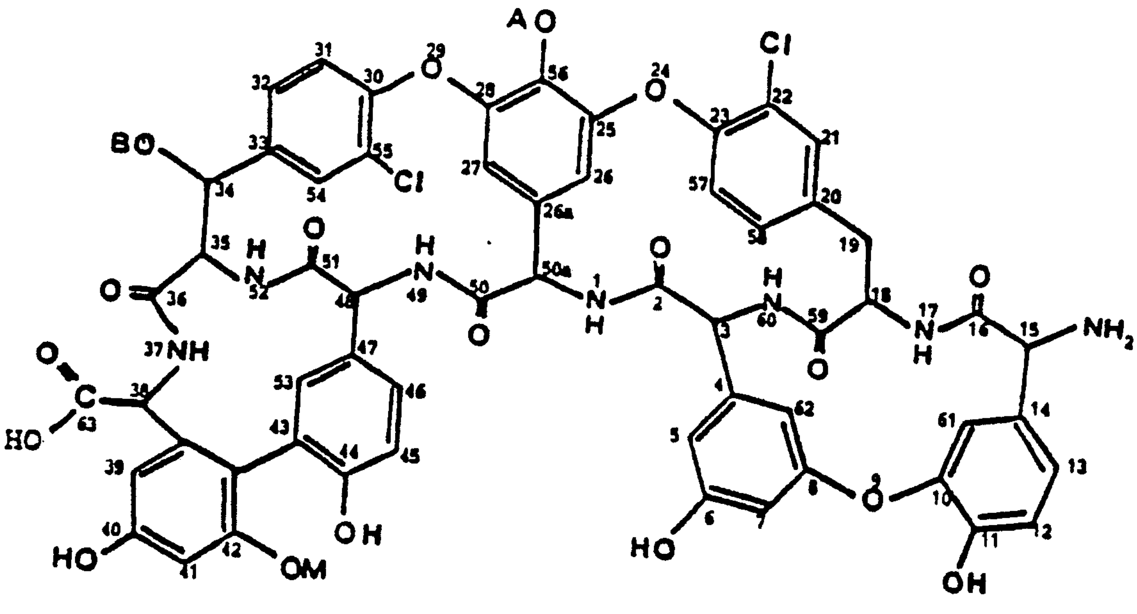

III

wherein A, B and M are defined as above with an alkyl halide $R^1$(halo) wherein the alkyl portion is represented by an "alkyl" group which corresponds to the desired meaning of $R^1$ in the final product and the halide is preferably a chloride, bromide or iodide and most preferably bromide or chloride.

The reaction occurs at about room temperature in the presence of an organic or inorganic base of medium strength such as triethylamine, trimethylamine, tributylamine, sodium or potassium bicarbonate and the like.

Preferably, the reaction medium includes also an organic solvent capable of at least partially solubilizing the starting materials such as organic amides, alkyl ethers, ethers of glycols and polyols, phosphoramides, sulfoxides and aromatic compounds e.g., dimethylformamide, dimethoxyethane, hexamethylphosphoramide, dimethylsulfoxide, benzene, toluene and mixtures thereof.

This solvent should have a low water content (generally below about 3%).

The alkyl halide and the starting teicoplanin derivative are preferably employed in about equimolecular proportion or in a slight molar excess of the alkyl halide so that the mono-alkyl derivative is obtained in good yields.

As already said, the reaction occurs at about room temperature so that external heating is not in general necessary, even if the reaction can be carried out at temperatures of 40-70 °C.

When a dialkyl derivative is desired wherein $R^1$ and $R^2$ are as defined above the mono-alkyl derivative having the $R^1$ substituent is prepared and then it is reacted with the suitable alkyl halide of formula $R^2$(halo) under essentially the same conditions described above for the mono-alkylation.

Alternatively, some particular compounds of the invention can be prepared by reductive alkylation. In this case, the teicoplanin starting material is reacted with a molar excess of a carbonylic compound having the same skeleton of the substituent $R^1$ and whose oxo group is on the carbon atom which is to link to the $N^{15}$amino group of teicoplanin moiety, in the presence of a suitable reducing agent, like as an alkali metal borohydride.

It goes without saying that the reductive alkylation process is not always possible but it depends on the characteristics of the starting carbonyl compound used.

A skilled chemist can easily recognize which reactant cannot be used in the preparation of the $N^{15}$ alkyl substituent of the invention by a reductive alkylation process.
For example, either a reactant having groups which can give undesired cross-reactions with the alkali metal borohydride or a reactant which is not stable because of the simultaneous presence of the essential oxo group and another interfering functional group, cannot be used.

The reductive alkylation process can be preferably used, for instance, when $R^1$ represents an alkyl chain having functional groups such as ethers, hydroxy or alkyl substituted amines.
In particular, when it is possible, in order to obtain the mono-alkyl derivative with better yields by means of the alkylation reductive process a preferred procedure is the following:

a) adding to a suspension of teicoplanin compound in an organic polar protic solvent a suitable

amount of an alkali metal borohydride at a temperature comprised between 0 °C and 30 °C, then

b) adding a molar excess of a carbonyl compound of at least 20 times with respect to teicoplanin starting material, and

c) reacting the resulting solution with an alkali metal borohydride.

Among the polar protic solvents lower alkanols having from 1 to 4 carbon atoms are preferred. In particular, methanol is the preferred solvent used.

Sometimes, in particular cases, a small amount of a polar protic co-solvent can be added to completely dissolve teicoplanin starting material during the course of the reaction, e.g. N-N-dimethylformamide, 1,3-dimethyl-3,4,5,6-tetrahydro 2(1H)-pyrimidone (DMPU), dimethylsulfoxide.

The temperature at which the reaction is carried out is comprised between 0 °C and 30 °C, preferably it ranges from 10 °C to 15 °C.

It is important to use a large molar excess of carbonyl compound, generally from 20 to 100 times with respect to teicoplanin starting material.

Sodium borohydride is the preferred alkali metal borohydride used in the step a).

As alkali metal borohydride in step c) the sodium borohydride is the preferred one, but also the potassium and the sodium cyano-borohydride can be used.

A preferred feature of this procedure is that of maintaining the reactants addition order as indicated above in steps a), b) and c).

In a preferred embodiment of the invention before adding the excess of carbonyl compound to the reaction mixture, the alkali metal borohydride is allowed to completely decompose under stirring. Generally it takes a time comprised between 30 minutes and 120 minutes, depending on the lower alkanol used as solvent.

In particular, the carbonyl compound can be preferably added when the reaction mixture is a clear solution.

The suitable amount of alkali metal borohydride added can be different depending on the particular teicoplanin compound used as starting material, on the solvent used and on the temperature of the reaction, but it is advisable to add an amount of alkali metal borohydride such that the pH of the reaction mixture is alkaline, preferably between pH 8 and 10, as determined after diluting a sample of the non-aqueous reaction mixture with 4 volumes of water.

The reaction times for preparing $N^{15}$-mono-alkyl derivatives of teicoplanin depend on the factors reported above such as the starting compound used, the solvent, the amount of alkali metal borohydride used and the like, and on the structure of reactant carbonyl compounds but, in general, it is preferred to leave the reaction mixture under stirring from 1 to 10 h after the addition of the reactants and before treating with the reducing agent.

It is also possible to prepare a di-alkyl derivative wherein $R^2$ is a $(C_1$-$C_3)$alkyl by reductive alkylation, starting from the monoalkyl teicoplanin derivative having the substituent $R^1$ as defined above. Also in this case it is necessary that the borohydride agent does not react with the functional groups which can be present in the alkyl chain of the substituent $R^1$. A process which can be preferably used comprises adding to a suspension of a teicoplanin compound having the substituent $R^1$ defined above without any functional group which can react with a borohydride agent, in an organic polar protic solvent a molar excess of a carbonyl compound having the same carbon skeleton of the substituent $R^2$ above and whose oxo group is on the carbon atom which is to link the $N^{15}$-amino group of the teicoplanin moiety, said molar excess being of at least 30 times with respect to the teicoplanin starting material, in the presence of an organic acid of such strength and in such amount that a sample of the obtained mixture diluted with four volumes of water shows a pH between 2.5 and 4, said acid being further characterized in that, under the reaction conditions, it cannot yield a reduction product which is a competitor of the carbonyl compound reactant in the reaction with the $N^{15}$ amino group of the teicoplanin moiety, and reacting the resulting mixture with a metal alkali borohydride at a temperature between 0 °C and 60 °C.

Among the organic polar protic solvents, alkyl alcohols having from 1 to 4 carbon atoms are preferred. Examples of alcohols which can be used are methanol, ethanol, propanol and butanol. In particular, methanol is preferred.

Sometimes in particular cases, a small amount of a polar co-solvent can be added to completely dissolve teicoplanin starting material during the course of the reaction, e.g. N,N-dimethylformamide, 1,3-dimethyl-3,4,5,6-tetrahydro-2(1H)-pyrimidone (DMPU), dimethylsulfoxide.

As alkali metal borohydride, sodium borohydride is preferably used, but also potassium and sodium cyano-borohydrides can be conveniently used.

The temperature can be preferably maintained between 30 °C and 40 °C.

The molar excess of the reactants with respect to teicoplanin ranges preferably from 30 to 300 times for

aldehydes or ketones.

The organic acid which is used in the present invention is characterized by having such a strength to show a pH between 2.5 and 4 when a sample of the reaction mixture is added to 4 volumes of water before adding the reducing agent. Furthermore, said acid must not negatively interfere in the reaction course. It means that it preferably has not to be reduced by the reducing agent employed ($NaBH_4$ and the like) or, if a reaction occurs, the product of this reaction is not a competitor with the carbonyl compound choosen as starting material.

The term "competitor" as used in specification and claims means any group which can be linked to an amino group with a bond like a "Schiff base" and subsequently reduced to an alkyl moiety, being said group different from the carbonyl compound used as starting material. Thus, in a preferred embodiment of the present invention when an aldehyde is used as the carbonyl compound the suitable acid to be preferably used is the carboxylic acid corresponding to said aldehyde; for example, formic acid with formaldehyde, acetic acid with acetaldehyde and so on. In the case that a keto compound (acetone, methyl ethyl ketone and the like) is used as the starting material, a preferred acid can be choosen among a ($C_1$-$C_6$)alkyl sulfonic acid and a ($C_6$-$C_{10}$)aryl sulfonic acid such as for example paratoluensulfonic acid, methanesulfonic acid and the like. The amount of acid used depends on several factors such as the teicoplanin starting material and the carbonyl compound but usually the desired pH is obtained when a molar excess of acid is used from 5 to 20 times with respect to teicoplanin.

As mentioned above it will be advisable to use the N-alkylation process instead of the reductive alkylation one depending on the particular compound which is desired according to the specific chemical features of the alkyl substituent.

In addition, the sugar moiety of an alkyl compound of formula I may be selectively removed to transform it into another alkyl compound of formula I.

For example, an alkyl compound of formula I wherein A, B, and M represent a sugar moiety as above defined can be transformed into the corresponding compound wherein B and M are as above and A is hydrogen by means of controlled acid hydrolysis in a strong concentrated aqueous organic acid.

The concentrated organic acid in this case is preferably aqueous trifluoroacetic acid at a concentration between 75% and 95%, and the reaction temperature is preferably between 10° and 50° C.

The preferred hydrolysis conditions are represented by about 90% trifluoroacetic acid at room temperature.

The reaction time varies depending on the other specific reaction parameters but, in any case, the reaction may be monitored by TLC or preferably HPLC techniques.

An analogous selective hydrolysis is reported in European Patent Application Publication No. 146822.

Similarly, alkyl compounds of formula I wherein A, B, and M represent a sugar moiety as above defined or A represents hydrogen and B and M represent sugar moieties as above defined can be transformed into the corresponding alkyl compounds of formula I wherein A and M represent hydrogen and B represents a sugar moiety as defined by means of a selective hydrolysis with a strong acid in the presence of a polar aprotic solvent selected from ethers, ketones, and mixture thereof which are liquid at room temperature.

Preferred hydrolysis conditions are in this case represented by the use of a concentrated mineral acid in the presence of an ether such as dimethoxyethane at room temperature.

Also in this case, the reaction course may be monitored by TLC or preferably HPLC. An analogous selective hydrolysis is reported in European Patent Application Publication No. 175100.

According to another embodiment of the present invention, an alkyl compound of formula I wherein A, B and M represent sugar moieties as defined above, or an alkyl compound of formula I wherein A and M represent hydrogen, and B represents a sugar moiety as above defined may be transformed into the corresponding alkyl compound of formula I wherein A, B and M represent hydrogen atoms by means of a selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly mixable with water, phenyl-substituted lower alkanols wherein the phenyl moiety may optionally carry ($C_1$-$C_4$)alkyl, ($C_1$-$C_4$)alkoxy or halo rests which at the reaction temperature are liquids slightly mixable with water, and beta-polyhalogenated lower alkanols, which at the reaction temperature are liquids; in the presence of a strong acid, compatible with the solvent, selected from strong mineral acids, strong organic acids and strong acid cation exchanger resins in the hydrogen form and at a temperature between 20° C and 100° C.

In this case, the preferred hydrolysis conditions are represented by the use of a mineral acid, such as hydrochloric acid, in an haloalkanol such as trifluoroethanol, at a temperature between 65° C and 85° C.

Analogous selective hydrolysis conditions on a similar substrate are described in European Patent Application Publication No. 146053.

In the following table (Table I) the structure formulas of representative examples of compounds of the invention are reported.

TABLE I

| COMPOUND | A | B | M | $R^1$ | $R^2$ |
|---|---|---|---|---|---|
| 1 | $-GNHCOR_{(1-5)}$ | $-GNHCOCH_3$ | -M | 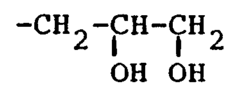 | H |
| 2 | do | do | do | 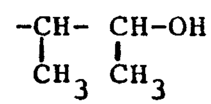 | H |
| 3 | do | do | do | 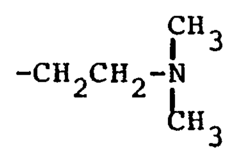 | H |

continued...

TABLE I

| COMPOUND | A | B | M | $R^1$ | $R^2$ |
|---|---|---|---|---|---|
| 4 | $-GNHCOR_{(1-5)}$ | $-GNHCOCH_3$ | -M | 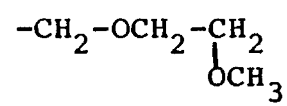 | H |
| 5 | H | H | H | 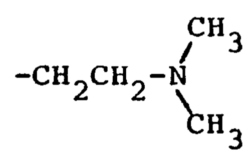 | H |
| 6 | $-GNHCOR_{(1-5)}$ | $-GNHCOCH_3$ | -M | 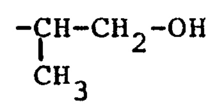 | H |

TABLE I continued...

| COMPOUND | A | B | M | $R^1$ | $R^2$ |
| --- | --- | --- | --- | --- | --- |
| 7 | H | H | H | $-CH(CH_3)-CH_2OH$ | H |
| 8 | do | do | do | $-CH(CH_3)-CH(CH_3)OH$ | H |

## HPLC Analysis

The following table (Table II) reports the $t_R$ of representative examples of the compounds that can be prepared according to the process of the invention.

The assays were run with a HPLC Hewlett - Packard 1090 apparatus equipped with UV detector (254 nm).

Columns:

Hypersil OD5 (silanized Silica gel, 5 micrometer) having dimension 100 x 4.6 mm of Hewlett-Packard.
Flow rate: 1.5 ml/min
Injection: 10 microliter

Eluents:

Solution A: Buffer 0.02 M $NaHPO_4$ (pH 4.7)
Solution B: $CH_3CN$

Gradient profile:

| minute: | 0 | 40 | 45 | 48 | 55 | 56 |
|---|---|---|---|---|---|---|
| % B in A | 10 | 33 | 47 | 47 | 10 | - |

TABLE II

| (HPLC Analysis) | | |
|---|---|---|
| No. COMPOUND | $t_R$ | $t_R/t_R$ L 17392 |
| 1 | 26.12 | 1.85 |
| 2 | 29.0 | 2.18 |
| 3 | 28.29 | 2.0 |
| 4 | 32.70 | 2.33 |
| 5 | 18.71 | 1.32 |
| Deglucoteicoplanin (standard) | 15.30 | 1.00 |

The following table (TABLE III) reports the acid-base titration data of some representative compounds of the invention. The assays were carried out in Methylcellosolve $^R/H_2O$, 4/1 (v/v). A sample (10 micromole) is dissolved in about 20 ml, then 0.01 N HCl (2 ml) is added and the mixture is titrated with 0.01 N NaOH in the same solvent mixture.

TABLE III

| Acid base titration | | | | |
|---|---|---|---|---|
| COMPOUND | Salt Form | $pK_1$ | $pK_2$ | $pK_3$ |
| 1 | $C_{91}H_{103}O_{35}N_9Cl_2$ 1) | 4.7 | 6 | - |
| 2 | $C_{92}H_{105}O_{34}N_9Cl_2$ 1) | 5.05 | 6.35 | - |
| 3 | $C_{92}H_{106}O_{33}N_{10}Cl_2$ 1) | 4.85 | 6.2 | 8.45 |
| 5 | $C_2H_{54}O_8N_8Cl_2$ | 4.75 | 6.5 | n.d.2) |

1) Related to the peak corresponding to the derivative of teicoplanin $A_2$ component 2
2) Hidden by phenol groups

Table IV reports $^1$H-NMR data obtained at 250 MHz with a Bruker AM-250 Spectrometer in DMSO-$D_6$ at 20 °C, at a sample concentration of 20 mg/ml (internal standard : TMS, delta = 0.00 ppm).

TABLE IV

| $^1$H-NMR Spectra (delta, ppm; multiplicity, J, $H_z$) | |
|---|---|
| Compound 1: | 0.79, 1.0-1.22, 1.40, 2.02 (acyl chain); 1.86 (acetylglucosamine); 2.80-3.70 ($CH_2OH$ and $CH_2$-N); 4.08-5.60 (peptidic CH'S); 6.29-7.80 (aromatic CH's) |
| Compound 2: | 0.84, 1.11-1.17, 1.42, 2.00 (acyl chain); 1.87 (acetylglucosamine); 0.98-1.10 [$CH_3$-(CH)]; 4.10-5.68 (peptidic CH'S); 6.14-7.91 (aromatic CH's) |
| Compound 3: | 0.84, 1.10-1.17, 1.42, 1.99 (acyl chain); 1.87 (acetylglucosamine); 2.21 [$N(CH_3)_2$]; 4.09-5.70 (peptidic CH's); 6.20-7.94 (aromatic CH's) |
| Compound 4: | 0.84, 1.11-1.17, 1.42-2.02 (acyl chain); 1.89 (acetylglucosamine); 3.20 ($OCH_3$); 3.68 ($OCH_2$); 4.06-5.66 (peptidic CH's); 6.20-7.88 (aromatic CH's) |
| Compound 5: | 2.79 [$NH^+(CH_3)_2$] ; 3.2-3.4 ($CH_2$-$CH_2$); 4.11-5.66 (peptidic CH's); 6.23-7.78 (aromatic $CH_3$) |

The antibacterial activity of the compounds of the present invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37° C. The results of the in vitro antibacterial testing of representative compounds of formula I are summarized in Table V.

TABLE V

*In vitro* (MIC microgram/ml)

| Test Organism | | Compound No. 1 | Compound No. 2 | Compound No. 3 |
|---|---|---|---|---|
| *S. haemolyticus* L 602 | | 32 | 16 | 2 |
| *S. aureus* Tour $10^4$ | | 0.5 | 0.5 | 0.125 |
| *S. aureus* Tour $10^6$ | | 2 | 1 | 0.5 |
| *S. aureus* Tour + 30% bovine serum | | 2 | 2 | 0.5 |
| *S. epidermidis* ATCC 12228 | | 0.25 | 0.25 | 0.125 |
| *S. pyogenes* C203 | | 0.06 | 0.06 | 0.125 |
| *S. pneumoniae* UC41 | | 0.25 | 0.25 | 0.125 |
| *S. faecalis* ATCC 7080 | | 0.12 | 0.12 | 0.125 |
| *E. coli* SKF 12140 | | >128 | >128 | >128 |
| *P. vulgaris* X19H ATCC 881 | | >128 | >128 | >128 |
| *P. aeruginosa* ISM | | >128 | >128 | >128 |
| *In vivo* (mice) | s.c. | - | - | 0.41 |
| $ED_{50}$ (mg/kg) *S. pyogenes* L 49 | o.s. | - | - | >300 |

(Continued) TABLE V

In vitro (MIC microgram/ml)

| Test Organism | | Compound No. 4 | Compound No. 5 | Compound No. 6 |
|---|---|---|---|---|
| S. haemolyticus L 602 | | 8 | 0.5 | 1.6 |
| S. aureus Tour $10^4$ | | 0.12 | 0.125 | 0.25 |
| S. aureus Tour $10^6$ | | 0.5 | 0.125 | 1 |
| S. aureus Tour + 30% bovine serum | | 1 | 1 | 2 |
| S. epidermidis ATCC 12228 | | 0.125 | 0.06 | 0.5 |
| S. pyogenes C203 | | 0.06 | 0.25 | 0.12 |
| S. pneumoniae UC41 | | 0.125 | 0.125 | 0.25 |
| S. faecalis ATCC 7080 | | 0.125 | 0.25 | 0.12 |
| E. coli SKF 12140 | | >128 | >128 | >128 |
| P. vulgaris X19H ATCC 881 | | >128 | >128 | >128 |
| P. aeruginosa ISM | | >128 | >128 | >128 |
| In vivo (mice | s.c. | - | 1.25 | - |
| $ED_{50}$ (mg/kg) S. pyogenes L 49 | o.s. | - | - | - |

(Continued) TABLE V

In vitro (MIC microgram/ml)

| Test Organism | Compound No. 7 | Compound No. 8 |
|---|---|---|
| S. haemolyticus L 602 | 0.5 | 0.5 |
| S. aureus Tour $10^4$ | 0.06 | 0.12 |
| S. aureus Tour $10^6$ | 0.12 | 0.12 |
| S. aureus Tour + 30% bovine serum | 1 | 2 |
| S. epidermidis ATCC 12228 | 0.06 | 0.06 |
| S. pyogenes C203 | 0.12 | 0.12 |
| S. pneumoniae UC41 | 0.12 | 0.25 |
| S. faecalis ATCC 7080 | 0.25 | 0.12 |
| E. coli SKF 12140 | >128 | >128 |
| P. vulgaris X19H ATCC 881 | >128 | >128 |
| P. aeruginosa ISM | >128 | >128 |
| In vivo (mice) | s.c. 0.84 | 0.86 |
| $ED_{50}$ (mg/kg) S. pyogenes L 49 | o.s. - | - |

The $ED_{50}$ values (mg/Kg) of representative compounds of the invention in vivo tests in mice experimentally infected with S. pyogenes L 49 and reported in Table V were carried out according to the procedure described by V. Arioli et al., Journal of Antibiotics 29, 511 (1976).

In view of the above reported antimicrobial activity, the compounds of the present invention can effectively be employed as the active ingredient of antimicrobial preparations used in human and veterinary medicine for the prevention and treatment of infectious diseases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage froms. Preparations for oral administration may be in the form of capsules, tablets, liquid solutions or suspensions. As known in the art the capsules and tablets may

contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms for absorption through the mucous membranes of the nose and throat or bronchial tissues and may conveniently take the form of liquid sprays or inhalants, lozenges, or throat paints.

For medication of the eyes or ears, the preparation may be presented in liquid or semi-liquid form. Topical applications may be formulated in hydrophobic or hydrophilic bases as ointment, creams, lotions, paints, or powder.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compound of the invention are generally effective at a dosage comprised between about 0.5 and about 30 mg of active ingredient per Kg of body weight, preferably divided in 2 to 4 administrations per day. Particularly desirable compositions are those prepared in the form of dosage units containing from about 20 to about 300 mg per unit.

A parenteral solution is prepared with 100 mg of compound 3 of Example 4 dissolved in 1.5 ml of sterile water for injection.

A parenteral solution is prepared with 250 mg of compound 1 or 5 hydrochloride dissolved in 2 ml of sterile water for injection.

A topical ointment is prepared with 200 mg of compound 1 or 5
3.6 g of polyethylene glycol 4000 U.S.P.
6.2 g of polyethylene glycol 400 U.S.P.

Besides their activity as medicaments, the compounds of the present invention can be used as animal growth promoters.

For this purpose, one or more of the compounds of the invention is administered orally in a suitable feed. The exact concentration employed is that which is required to provide for the active agent in a growth promotant effective amount when normal amounts of feed are consumed.

The addition of the active compounds of the invention to animal feed is preferably accomplished by preparing an appropriate feed premix containing the active compounds in an effective amount and incorporating the premix into the complete ration.

Alternatively, an intermediate concentrate or feed supplement containing the active ingredient can be blended into the feed.

The way in which such feed premixes and complete rations can be prepared and administered are described in reference books (such as "Applied Animal Nutrition", W.H. Freedman and Co., S. Francisco, USA, 1969 or "Livestock Feeds and Feeding", O and B Books, Corvallis, Oregon, USA, 1977) and are incorporated herein by references.

The following examples further illustrate the invention and must not be construed as limiting it:

## Example 1

$N^{15}$-(2,3-Dihydroxy)propyl teicoplanin (compound 1)

To a stirred suspension of teicoplanin (6 g, 3 mmol) in methanol (180 ml), $NaBH_4$ pellet (6 g, 157 mmol) is added maintaining the reaction temperature at about 20°C. The mixture is stirred at room temperature for 2 hours then D,L-glyceraldehyde (5 g, 55 mmol) is added. The reaction is continued overnight at the same

temperature, then a second portion of glyceraldehyde (5 g, 55 mmol), followed after 1 hour by $NaBH_4$ (6 g) is added and the reaction continued for two hours. The mixture is diluted with methanol (120 ml), cooled to 5°C and $NaBH_4$ pellets (5 g) is added maintaining the temperature at about 30°C. The mixture, adjusted to pH 5 with acetic acid, is concentrated under vacuum and the oily residue which forms is dissolved in 400 ml of water and the resulting solution is loaded at the top of a column of 600 g of silanized silica-gel (0.06-0.2 mm; Merck Co.). The column is eluted before with 3 l of water then with $CH_3CN/H_2O/CH_3COOH$ (45/45/10) and fractions are analyzed by HPLC. The fractions are combined according to their content, diluted with n-butanol and evaporated under vacuum at 50°C. The residue is treated with ether, collected by filtration and dried, yielding 4.5 g of compound 1.

Example 2

$N^{15}$-(1-methyl-2-hydroxy)propyl) teicoplanin (compound 2)

By following the procedure of example 1 but using acetoin (5 g; 57 mmol) instead of D,L-glyceraldehyde as the carbonyl compound, 4.9 of compound 2 is obtained starting from teicoplanin (6 g, 3 mmol) .

Example 3

$N^{15}$-(2-dimethylamino)ethyl teicoplanin aglycon (compound 5)

To a suspension of teicoplanin aglycon (2 g, 1.6 mmol) cooled to 5°C, $NaBH_4$ (3.8 g, 100 mmol) is added under a nitrogen atmosphere, while keeping the reaction temperature at about 40°C. The mixture is stirred 40 minutes then dimethylamino acetaldehyde hydrochloride (1.5 g, 13.5 mmol) dissolved in methanol (20 ml) is added (dimethylamino acetaldehyde hydrochloride is obtained from dimethylaminoacetaldehyde diethyl-acetal (Aldrich) by acidic hydrolysis according to J.H. Biel; W.K. Hoya and H.A. Leiser; J.A.C.S. 81, 2527; 1959). The reaction mixture is stirred two hours at room temperature, then cooled to 10°C, and a second portion of $NaBH_4$, (1.2 g, 31.7 mmol) is added thereto. The HPLC analysis after one hour shows that 30% of the starting antibiotic is still present. Dimethylamino aldehyde hydrochloride (0.5 g, 4.5 mmol) and $NaBH_4$ (600 mg, 15.7 mmol) are then added. After standing overnight the reaction is worked up by cooling the reaction mixture to 0°C, adjusting to pH 6 with 10% (w/w) HCl, and concentrating under reduced pressure.
The crude material is suspended in water and loaded on a chromatographic column containing 100 g of silanized silica gel that is sequentially eluted with water (500 ml), 15% (w/w aqueous) $CH_3CN$ (500 ml) and a gradient $CH_3CN/H_2O$ from 15% to 25% acetonitrile in water. The fractions containing the antibiotic of the title (HPLC) are combined and concentrated under vacuum and the residue is treated with ethyl ether, collected by filtration and dried to give 1.050 g of compound 5.

Example 4

$N^{15}$-(2-dimethylamino)ethyl teicoplanin (compound 3)

By essentially following the procedure of example 3 but using teicoplanin instead of teicoplanin aglycon as the starting material, 1.120 g of compound 3 are obtained.

Example 5

$N^{15}$-(2-methoxyethoxy)methyl teicoplanin (compound 4)

A solution of teicoplanin (2 g, 1 mmol) and $NaHCO_3$ (56 mg, 0.066 mmol) in DMF (120 ml) is heated for

15 minutes at 40 °C, then triethylamine (TEA) (1.2 ml, 8.6 mmol) and 2-methoxyethoxymethylchloride (MEM chloride, Aldrich) (0.56 ml, 4.9 mmol) are successively added. The reaction mixture is heated at 50 °C for two hours then DMF is evaporated under vacuum and the residue is dissolved in n-butanol/water, 150 ml/70 ml. The organic phase is separated, washed with water (50 ml) and the solvent is evaporated under vacuum. The residue, dissolved in $CH_3CN/H_2O$ 30/70 is purified on a silanized gel column as described in example 3 but with a linear gradient from 30% to 50% of $CH_3CN$ in $H_2O$, yielding 500 mg of pure compound n. 4.

Example 6

$N^{15}$-(1-methyl-2-hydroxy)ethyl teicoplanin (compound 6)

By essentially following the procedure of example 1 but using 3-hydroxy-2-propanone instead of D,L glyceraldehyde as carbonyl compound, 4 g of compound 6 are obtained starting from teicoplanin (6 g, 3 mmol) .

Example 7

$N^{15}$-(1-methyl-2-hydroxy)ethyl teicoplanin aglycon (compound 7)

By substantially following the procedure of example 1 but using 3-hydroxy-2-propanone instead of D,L-glyceraldehyde as the carbonyl compound and teicoplanin aglycon instead of teicoplanin as the starting antibiotic material, 4 g of compound 7 are obtained.

Example 8

$N^{15}$-(1-methyl-2-hydroxy)propyl teicoplanin aglycon (compound 8)

By essentially following the procedure of example 2 but using teicoplanin aglycon instead of teicoplanin as the antibiotic starting material 4,7 g of compound 8 are obtained.

**Claims**

1. $N^{15}$-alkyl and $N^{15}$,$N^{15}$-di-alkyl derivatives of teicoplanin compounds having the following formula:

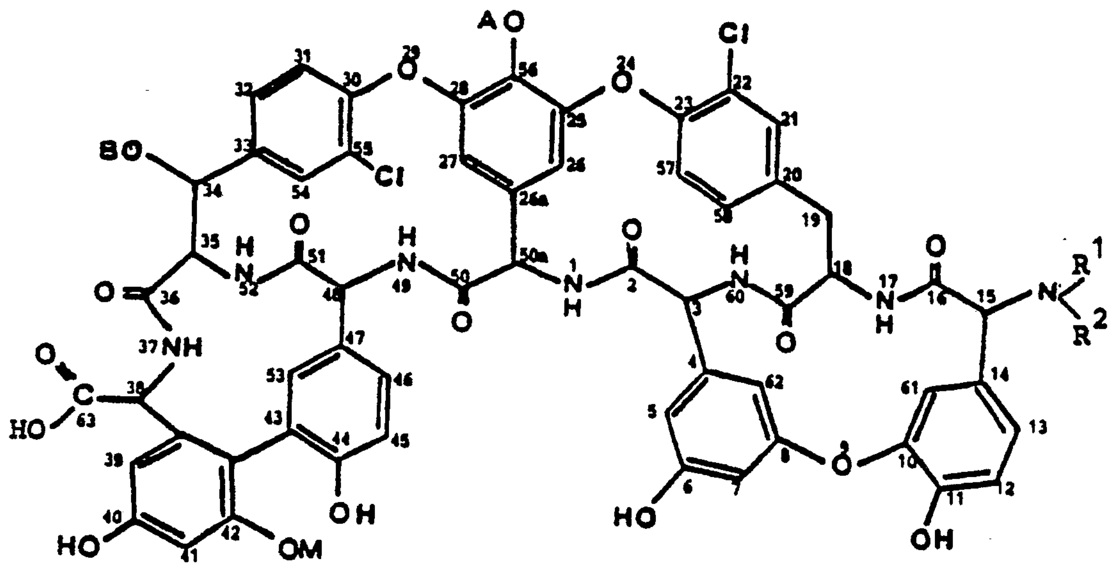

I

wherein:

$R^1$ represents

$[CHR^3(CR^4R^5)_mX]_p-(CH_2)_n-R^6$     II

wherein $R^3$ and $R^4$ independently represent H or a $(C_1-C_6)$ alkyl; $R^5$ represents H, a $(C_1-C_6)$alkyl or OH; $R^6$ represents H, $COOR^7$, $S(R^7)$; $N(R^7R^8)$, $OR^7$, halogen or a $(C_1-C_3)$alkyl; $R^7$ and $R^8$ independently represent H, a $(C_1-C_3)$alkyl; m, n and p are integers wherein m is zero or 1,$0 \leq n \leq 6$, $1 \leq p \leq 6$; X is O, NH or a bond with the proviso that when X is O or NH, $n \neq 0$, $1 \leq p \leq 3$ and $R^5$ is different from OH; $R^2$ represents hydrogen or a $(C_1-C_3)$alkyl group; with the further proviso that $R^1$ must not represent a $(C_1-C_{12})$alkyl group

A represents hydrogen or -N[$(C_9-C_{12})$aliphatic acyl]-beta-D-2-deoxy-2-amino-glucopyranosyl;

B represents hydrogen or N-acetyl-beta-D-2-deoxy-2-amino-glucopyranosyl;

M represents hydrogen or alpha-D-mannopyranosyl; with the proviso that B represents hydrogen only when A and M are simultaneously hydrogen; and the addition salts thereof,

2. A compound of claim 1 wherein

$R^1$ represents

$[CHR^3(CR^4R^5)_mX]_p-(CH_2)_nR^6$     II

wherein: $R^3$ and $R^4$ independently represent H or a $(C_1-C_4)$alkyl; $R^5$ represents a H, $(C_1-C_4)$alkyl or OH; $R^6$ represents H, $COOR^7$, $S(R^7)$ $N(R^7R^8)$; $OR^7$, halogen or a $(C_1-C_3)$alkyl; $R^7$ represents H or a $(C_1-C_3)$alkyl; m, n and p are integers wherein m is zero or 1, $0 \leq n \leq 3$, $1 \leq p \leq 3$, X is O, NH or a bond with the proviso that when X is O or NH, $n \neq 0$ and $R^5$ is different from OH; $R^2$ represents hydrogen or a $(C_1-C_3)$alkyl group with the proviso that $R^1$ must not represent a $(C_1-C_{12})$alkyl group

3. A compound of claim 1 wherein $R^1$ represents formula I wherein: $R^3$ and $R^4$ independently represent H or a $(C_1-C_4)$alkyl; $R^5$ represents H, $(C_1-C_4)$alkyl or OH; $R^6$ represents H, $S(R^7)$; $N(R^7R^8)$, $OR^7$; $R^7$ and $R^8$ independently represent H or a $(C_1-C_3)$alkyl; m, n and p are integers wherein m is zero or 1, $0 \leq n \leq 3$, $1 \leq p \leq 3$, X is O or a bond with the proviso that when X is O, $n \neq 0$ and $R^5$ is different from OH; $R^2$ represents H or a $(C_1-C_3)$alkyl group.

4. A compound of claim 1 wherein $R^1$ represents a group in which n ranges from 0 to 3, p ranges from 1 to 3, $R^6$ is $OR^7$ or N $R^7R^8$ m is zero or 1, X is O NH or a bond, $R^3$ and $R^4$ independently represent H or a $(C_1-C_6)$alkyl group, $R^5$ is H, a $(C_1-C_6)$alkyl group, or OH, $R^2$ is H or a $(C_1-C_3)$alkyl group, and $R^7$ and $R^8$ are defined as in claim 1.

5. A compound of claim 1 wherein $R^1$ represents a group wherein $R^3$ and $R^4$ independently represent H or $CH_3$; $R^5$ represents H or OH; $R^6$ represents H, $OR^7$ or $NR^7R^8$; $R^7$ and $R^8$ independently represent H or $CH_3$; m is zero or 1, n is zero or 1, p is 1, X is O or a bond with the proviso that when X is O, n is 1 and $R^5$ is different from OH; and $R^2$ is H or $CH_3$.

6. A compound of claim 1 wherein $R^1$ represents a group selected from

$-(CH_2)_p\ SCH_3$;

$-(CH_2)_p\ SH$;

$-(CH_2)_p\ COOCH_3$;
$-(CH_2)_p\ COOH$;
$-(CH_2)_p\ NH_2$;
$-(CH_2)_p\ N(CH_3)_2$;
wherein $1 \leq p \leq 6$ and $R^2$ represents H or a $(C_1-C_3)$alkyl.

7. A process for preparing a compound of claims 1, 2, 3, 4, 5, 6 or 7 which comprises reacting a teicoplanin starting material or a compound of formula III

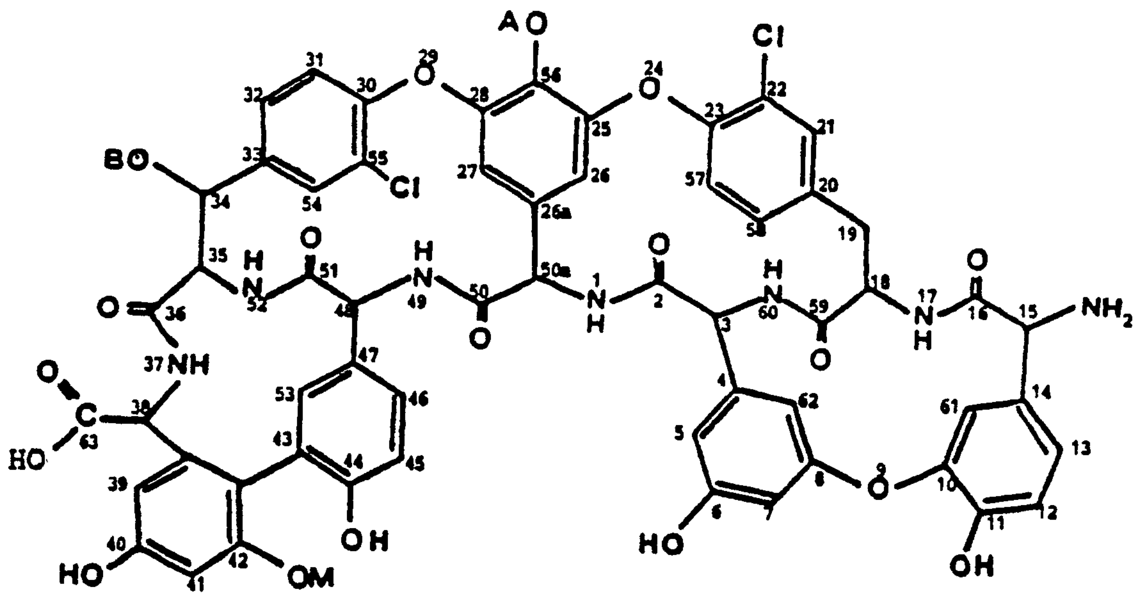

III

wherein A, B, M are as defined with an alkyl halide of formula $R^1$(halo) wherein $R^1$ is as defined and (halo) represents chloro or bromo in the presence of a base and possibly of an organic solvent.

8. The process of claim 7 wherein the teicoplanin starting material is selected from teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, compound A, compound B, antibiotic L 17054, antibiotic L 17046, antibiotic L 17382, or any mixture thereof in any proportion.

9. A process for preparing a compound of claims 1, 2, 3, 4, 5, 6 or 7 which comprises reacting a $N^{15}$-monoalkyl teicoplanin compound of formula I wherein $R^1$ is as defined above with an alkyl halide of formula $R^2$(halo) wherein $R^2$ is as defined and (halo) represents chloro or bromo in the presence of a base and possibly of an organic solvent.

10. A process for preparing a monoalkyl compound of claims 1, 2, 3, 4, 5, 6 or 7 which comprises:

a) adding to a suspension of a teicoplanin compound of formula III in an organic polar protic solvent a suitable amount of an alkali metal borohydride at a temperature comprised between 0 °C and 30 °C, then

b) adding a molar excess of a carbonyl compound of at least 20 times with respect to teicoplanin starting material, and

c) reacting the resulting solution with an alkali metal borohydride.

11. A process for preparing a dialkyl compound of claims 1, 2, 3, 4, 5, 6 or 7 which comprises adding to a suspension of a teicoplanin compound having the substituent $R^1$ defined above in an organic polar protic solvent a molar excess of a carbonyl compound having the same carbon skeleton of the substituent $R^2$ above and whose oxo group is on the carbon atom which is to link the $N^{15}$-amino group of the teicoplanin moiety, said molar excess being of at least 30 times with respect to the teicoplanin starting material, in the presence of an organic acid of such strength and in such amount that a sample of the obtained mixture diluted with four volumes of water shows a pH between 2.5 and 4, said acid being further characterized in that, under the reaction conditions, it cannot yield a reduction product which is a competitor of the carbonyl compound reactant in the reaction with the $N^{15}$ amino group of the teicoplanin moiety, and reacting the resulting mixture with a metal alkali borohydride at a temperature between 0 °C and 60 °C.

12. A pharmaceutical composition comprising a compound of claims 1, 2, 3, 4, 5 or 6 in admixture with a pharmaceutically acceptable acid.

13. Use of a compound of claim 1, 2, 3, 4, 5, or 6 for preparing a medicament for antimicrobial use.
14. A compound of claim 1, 2,3, 4, 5 or 6 for use as a medicine.